# EUROPEAN PATENT APPLICATION

(11) **EP 1 762 845 A2**
(43) Date of publication of application: **14.03.2007**
(21) Application number: 06396010.8
(22) Date of filing: 22.05.2006
(51) Int. Cl.: G01N 33/00

(54) **Method and device for prediction of moisture damage and drying**

(30) Priority: 24.05.2005 FI 20050550
(71) Applicant: Oy Pandes Ltd., 90630 Oulu (FI)
(72) Inventor: Salin, Risto, 90840 Haukipudas (FI)

(57) **Abstract**

Measuring method and device predicting humidity damage and drying, The measuring device comprises a humidity sensor encased in the floor and a separate central processor, which sensor and central processor have been interlinked via a cable or radio means. The measuring method measures humidity change by means of an angular coefficient for absolute humidity. The central processor emits an alarm if the angular coefficient accounting for the speed of humidity change exceeds the limit value.

## Description

### Field of the invention

The invention relates to measuring methods and more especially to measuring methods and devices predicting humidity damage and drying.

### Background to the invention

Humidity measurements have rapidly become more common in the last few years, especially in detached and serni-detached houses and blocks of flats. The trend is attributable to an increased awareness of humidity risks and leak damage that can cause extensive building damage and expose people to mildew and other microbes. Humidity damage is also common in public premises and day-care centres. According to surveys conducted by the National Public Health Institute, 82% of detached and semi-detached houses and 60% of blocks of flats have suffered from some form of humidity damage. The statistics of the Federation of Finnish Insurance Companies from 2003 show that leak damage was the only indemnification target showing considerably growth during the period 1983-2003. It is essential that the damage is detected and repaired fast, as humidity problems easily become aggravated and mildew damage can already develop in a couple of days.

Humidity measuring methods and the devices used in such measurements can be divided into different categories. Among the devices are equipment used in the processing industry and atmospheric measurements, which represent the most demanding category in terms of their technical properties. There are also methods and devices for measuring humidity in massive materials, such as building structures. Their technical properties are usually less advanced than those of the previously mentioned devices. Devices in the first category are typically used to carry out continuous measurements while those in the latter category are used for check-up measurements.

Humidity measuring methods are typically based on measuring the electrical properties of the material. Humidity changes the electric properties of material, which change can be observed using a humidity measuring device. Measuring methods and devices of this kind are typically based on capacitive or resistive measurements. Capacitive measuring is used e.g. in surface humidity meters by means of which structural humidity can be measured from the surface of the structure. In capacitive measuring, humidity changes the dielectric constant of the material, which changes the response of the electric circuit of the surface humidity meter. Another alternative is to measure the current passing through the material. In a resistive measuring method of this kind, in which method such alternative is used, humidity changes the electric resistance of the material and the change can be detected by means of the measuring device. With the measuring devices described above, it is possible to measure humidity inside the structure by opening the structure locally using a drill, for example. With this procedure, humidity deposited deeper in the structure can be detected more reliably than by measuring from the surface only.

One humidity measuring method currently in use is the wireless (RFID) humidity sensor, which is installed in the object to be measured. This allows long-term monitoring of humidity at the site without having to break the structure. The sensors can be installed in different parts of risk-susceptible premises and read by means of customised reading devices. The method in question is based on determining the material's dielectric constant at a high frequency.

Another humidity measuring method and device of the prior art measures relative humidity and temperature at the object. From these data the said method and device is capable of calculating the condensation temperature, absolute humidity, mixing ratio, wet bulb temperature and enthalpy. The device allows the scaling of measuring ranges and emits alarms if the measurement results exceed the set values. The device is intended for use in demanding applications employed in the processing industry.

Another humidity measuring method and device of the prior art automatically measures relative humidity and temperature at the object from the walls of the building (US 6377181 B1) during a time span of one day. The device is capable of emitting alarms if the measurement results exceed the set values.

However, there are several problems with the methods and devices of the prior art that are used to perform humidity measurements. They do not have the properties required of good measuring methods and devices that are used to predict humidity damage. In particular they have defects in communicating, to the body responsible for the condition of the building, information that predicts humidity damage. In addition, in automatic measuring methods of the prior art, methods predicting the drying of cast concrete are not in use.

An essential aspect in measuring humidity in real properties and predicting humidity damage is that humidity can be detected fast. In measuring methods of the prior art, damage cannot be detected predictably or in real time. In methods of the prior art, measurements are typically conducted once a year or when there are other visible reasons for carrying out the measurements, such as tiles coming off. This is a very serious defect, because damage can then develop into a state difficult to repair and become more aggravated. In addition, the lack of a low-cost, real-time measuring method hampers the broad adoption of humidity measurements and devices in buildings and building sites. The slow adoption of such measurements and devices is essentially due to the fact that the properties of the devices available on the market are inadequate, which gives rise to prejudice against humidity measurements.

The problem with surface humidity measurements, which are the most common measuring method, is the unreliability arising from the presence of reinforcements in the material, for instance. Surface humidity measurement is sensitive to tile surface humidity, which means that the premises to be measured should not be used e.g. for showering for a long time before the measurement. Major problems with this measuring method are the inaccuracy of the absolute result and other environment-related factors causing unreliability in the measuring method.

There are several problems with humidity measurements in which the structure has to be broken. This measuring method is also commonly used in real properties. In the method, the surface to be measured must be broken by means of a drill, for example. This means that water insulation, which is important to the functioning of the structure, has to be broken. As it is desired to measure humidity from critical places, having to break the structure is a major defect in this measuring method. In addiiion, the method sutfers from too large a volume of work, including the breaking and repair of structures. The drilled hole must be closed and humidity let to balance out for several days before reliable measurement results can be obtained. Another problem with the method is the lack of real time measurements.

There are also major defects with wireless (RFID) structural humidity measurements. In this measuring method, humidity measurement results cannot be obtained in real time. In this respect, it cannot be considered a predictive humidity damage measuring method at all. At the installation stage, the mounting of wireless sensors requires the use special plasters and an installation point must opened in the concrete for placing the sensor. These are major defects with regard to possibilities to apply the measuring method and device.

A major defect in a device of the prior art (US 6377181 B1) is that the device cannot be used for predictive measurements, because its use is only limited to changes taking place during one day. According to recent research results, it can take weeks or months for humidity damage to spread in floor structures so humidity changes then take place very slowly. The device is not capable of detecting changes of this kind. Another major defect in the device is that it is incapable of taking into consideration seasonal humidity changes taking place during a long measuring period, because the measurement is not essentially based on the measuring of absolute humidity. Relative humidity varies greatly during the year without any changes taking place in absolute humidity so a major defect in the device is that it can only be used to measure relative humidity. In addition, the device is only intended for measuring humidity in walls so it is incapable of predicting future humidity behaviour.

The most serious detect in humidity measuring methods of the prior art is that they do not make use of predictive calculation methods. With a predictive measuring method, it is possible to avoid the arising of humidity damage because even a small rise in humidity content can be detected in good time.

### Summary of the invention

The aim of the present invention is to develop a measuring method and device capable of predicting humidity damage in structures and the drying of structures in such a way that the defects caused by the aforementioned problems can be avoided.

The aims of the invention can be achieved by means of the present measuring method and device, which is characterised by what is said in the independent claims.

Some preferred embodiments of the invention are presented in the dependent claims.

The invention is based on measuring humidity in structures by means of a sensor, which is encased in the structure and which said sensor transmits constant measurement data to a central processor. Where necessary, the central processor converts to absolute humidity the relative humidity data that it has obtained from the sensor, and calculates from absolute humidity an angular coefficient that accounts for the speed of humidity change. The central processor monitors the angular coefficient for absolute humidity change and the level of relative humidity, emitting alarms if the measurement results exceed the set values.

The humidity measuring method and device according to the invention comprise a central processor and one or more humidity measuring sensors, which are connected to the central processor by means of a cable, radio means or by some other known means.

According to one preferred embodiment of the invention, the central processor is an electronic device, computer or automatic system capable of performing calculation operations.

According to another preferred embodiment, the sensor and central processor have been integrated into a single whole, in which case they denote the same physical device.

According to yet another preferred embodiment, some of the functions are provided in the sensor and some in the central processor.

According to still another preferred embodiment, the central processor is located in an easily accessible place on the room wall.

According to yet another preferred embodiment, the central processor calculates an angular coefficient for relative humidity or calculates, from the data it has obtained from the sensor, some other calculation result variable that is connected with a change of humidity in the structure.

According to still another preferred embodiment, the central processor provides a mean of the measurement results.

According to still another preferred embodiment, the central processor emits real time alarms on the basis of the measurement data. The alarms can be read from the display of the central processor or they are sent in the form of SMS messages, e-mail or by some other known means to a supervisor or to a monitoring system.

According to yet another preferred embodiment, the central processor registers measurement data from the sensor and calculates the said angular coefficient for humidity when the concrete for the structure has just been cast. The central processor provides a prediction of when the structure has dried up to the extent that the concrete can be coated.

The advantage of the measuring method and device according to the invention is that humidity damage is detected immediately when the structure begins to get wet. This crucial benefit has been made possible by means of a predictive method that calculates an angular coefficient for absolute humidity. It is thus possible to detect even slight humidity changes in the structure, eliminate the cause for humidity entering the structure and thereby completely avoid the arising of humidity damage that would require repair.

The advantage of the measuring method and device according to the invention is that humidity damage can be detected even if the resulting humidity change is small and takes places during a long time span. Still another advantage of the device is that it is capable of detecting and identifying humidity changes from the natural humidity variation that takes place during the year.

Another advantage of the invention is that the device can also emit a control command to an actuator on the basis of the measurement and calculation result so as to start e.g. a heater or blower.

Yet another advantage of the invention is that the device according to the invention can be easily and rapidly mounted in connection with the normal casting of the floor structure and does not have to be installed separately.

Yet another advantage of the invention is that the measuring method and device are capable of constantly monitoring their operation and reporting any fault state that may occur.

Still another advantage of the invention is that in fixed installation, the measuring environment remains the same, there are few disturbing factors and a relative change in humidity can be indicated with a very high accuracy.

Still another advantage of the invention is that when applying the measuring method, it is possible to use high-quality, accurate sensors, and structures need not be broken at any stage.

Still another advantage of the measuring method and device is that it can be used to constantly monitor and ensure the condition of buildings that are not otherwise monitored. Examples of this kind are public buildings in which leak damage causes major costs to the society and inconvenience to people using them.

Another advantage of the measuring method according to the invention is that the drying of the structure can be monitored and predicted so that the surface structure is mounted only when the humidity in the structure has dried up to the desired level. This will greatly reduce the risk that the builder faces if coating an excessively humid structure. In addition, the amount of work required at the building stage can be reduced when clear measurement data and prediction are available and indicate the time after which the structure can be coated safely.

### Brief description of the drawings

The invention will be explained in more detail below with regard to its preferred embodiments and with reference to the enclosed drawings, in which
- figure 1: shows an exemplary structure of the measuring device according to the invention, including the central processor and sensor,
- figure 2: shows an exemplary flow chart describing the functioning of the measuring method according to the invention.

### Detailed description of the invention

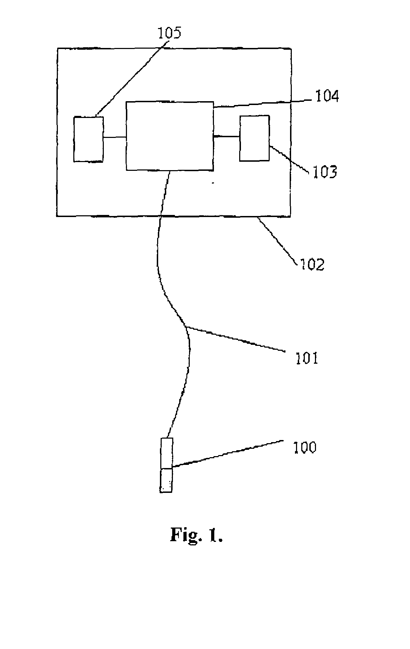

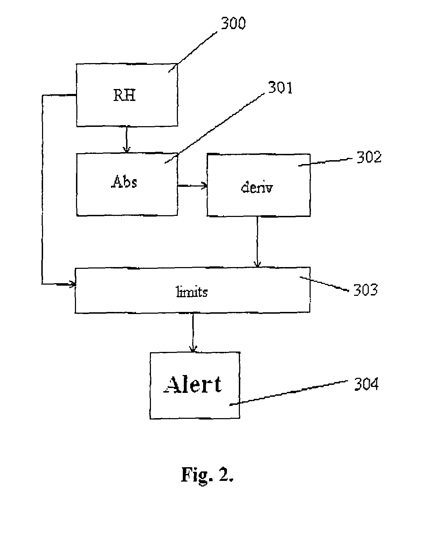

The structure of the measuring device, according to the invention, which said device predicts humidity damage and drying, will be explained below by referring to Figures 1 and 2. The structure of the device is shown schematically in the examples presented in Figures 1 and 2 and structural dimensions are indicated in such a way that the invention can be preferably illustrated. Thus the dimensions used in the figures do not reflect the actual dimensions of the device according to the invention.

The measuring device predicting humidity damage and drying is preferably a device that comprises a cast-mounted installation box 100, a cable or radio means 101 and a wall-mountable box 102. The mountable box 102 preferably comprises an intelligent part 104, keyboard 105 and display 103, which can be a led display or 7-segment display, for instance. The installation box 100 preferably comprises the actual box and a sensor element mounted inside the box. The mountable box 102 and installation box 100 are interlinked via connection 101, which connection can be a cable or wireless data transmission connection.

The operation of the measuring method according to the invention, which said method predicts humidity damage and drying, will be explained below with reference to figure 2, which shows the operation of the method schematically and in a way that the invention can be preferably illustrated. It should be noted that the functional diagram does not account for the overall operation of the device according to the invention.

The measuring method is preferably a method for measuring, by means of a sensor, the relative humidity (RH) 300 of the material. The data are converted to absolute humidity at stage 301 by means of a known formula. From the measurement data obtained at stage 301, for which data a mean is preferably provided, an angular coefficient is preferably calculated at stage 302. The said angular coefficient accounts for a rise in the amount of humidity in the material during a given time span. The measurement interval can be e.g. an hour, day, week, month or year, depending on the object of measurement. When measuring changes that take place slowly, the interval is essentially a week, month or year. The measured and calculated data are compared with the set limit values 303, which have preferably been set in such a way that any measurement results exceeding the set values predict the arising of humidity damage or reveal the presence of humidity damage. If the limit values are exceeded, an alarm, report or warning will he emitted at stage 304. Alarm data 304 are conveyed to the display of the device or transmitted further to the building automation system or as an SMS message, e-mail or by some other known means to the user or supervisor.

The measuring method and device, according to the invention, which said method and device predict humidity damage and drying, can be utilised especially in different types of structures used in bathrooms. The measuring method and device can also be used for measuring humidity in foundation, base floor, basement, roof and façade structures. It can also be used as a measuring method predicting the drying of newly cast concrete.

It is evident to those skilled in the art that as technology advances, the basic idea of the invention can be implemented in a variety of ways. Thus the invention and its embodiments are not limited to the aforementioned examples, but changes and modifications may be made therein without departing from the claims.

## Claims

1. A measuring method for predicting humidity damage or drying, **characterised in that** with the central processor (104) provided in the measuring arrangement, a variable (301, 302) indicating the speed of humidity change in at least one foundation, floor or roof structure is calculated on the basis of data obtained from at least one humidity sensor over a period of at least one week.

2. A measuring method according to claim 1, **characterised in that** the variable indicating the speed of humidity change is an angular coefficient for absolute humidity or an angular coefficient for water vapour subpressure.

3. A measuring method according to claim 2, **characterised in that** the central processor (104) can emit at least one alarm, report or warning of a reading (304) that exceeds the variable's limit value.

4. A measuring method according to claim 2, **characterised in that** the central processor (104) emits a control command to the actuator on the basis of the magnitude of the variable.

5. A measuring method according to claim 2, **characterised in that** when calculating the angular coefficient, a mean is employed that covers a period of one week, month, year or a corresponding period of time.

6. A measuring method according to claim 1, **characterised in that** the central processor (104) reports a prediction for the period after which the structure can be coated or after which the structure no longer has to be dried or after which humidity has reached the limit value.

7. A measuring arrangement predicting humidity damage and drying, **characterised in that** it comprises a central processor (104), which has been furnished to calculate, on the basis of information obtained from at least one sensor, which sensor has been embedded in the foundation, floor or roof structure to be examined and which sensor conveys data to the central processor, at least one variable (301, 302), which accounts for the speed of humidity change in the building structure over a period of at least one week.

8. Measuring arrangement according to claim 7, **characterised in that** the variable accounting for the speed of humidity change is an angular coefficient for absolute humidity or an angular coefficient for water vapour subpressure, which said coefficient is calculated from the values measured using the humidity sensor.

9. Measuring arrangement according to claim 7, **characterised in that** the central processor (104) has been furnished to emit at least one alarm, report or warning of a reading (304) that exceeds the variable's limit value.
